(19) **Europäisches Patentamt European Patent Office Office européen des brevets**

(11) **EP 1 257 703 B1**

(12) **EUROPEAN PATENT SPECIFICATION**

(45) Date of publication and mention of the grant of the patent:
**09.07.2008 Bulletin 2008/28**

(51) Int Cl.:
*D04H 1/42* (2006.01)          *D04H 1/54* (2006.01)
*A61F 13/15* (2006.01)

(21) Application number: **00986371.3**

(22) Date of filing: **14.12.2000**

(86) International application number:
**PCT/US2000/033852**

(87) International publication number:
**WO 2001/049912 (12.07.2001 Gazette 2001/28)**

(54) **BIODEGRADABLE THERMOPLASTIC NONWOVEN WEBS FOR FLUID MANAGEMENT**

BIOLOGISCH ABBAUBARE THERMOPLASTISCHE VLIESSTOFFE ZUR FLÜSSIGKEITSKONTROLLE

BANDES NON TISSEES THERMOPLASTIQUES BIODEGRADABLES DESTINEES A LA GESTION DES FLUIDES

(84) Designated Contracting States:
**DE ES FR GB IT TR**

(30) Priority: **29.12.1999 US 474634**

(43) Date of publication of application:
**20.11.2002 Bulletin 2002/47**

(73) Proprietor: **KIMBERLY-CLARK WORLDWIDE, INC. Neenah, Wisconsin 54956 (US)**

(72) Inventors:
• **TSAI, Fu-Jya, Daniel Appleton, WI 54915 (US)**

• **WERTHEIM, Brigitte, C. Appleton, WI 54914 (US)**

(74) Representative: **Davies, Christopher Robert Frank B. Dehn & Co. St Bride's House 10 Salisbury Square London EC4Y 8JD (GB)**

(56) References cited:
**EP-A- 0 801 172          WO-A-00/32245
US-A- 5 698 322          US-A- 5 759 569
US-A- 5 783 505**

## Description

### Technical Field

The invention relates to fluid management materials used in personal care products.

### Background of the Invention

As environmental awareness grows, the need for more environmentally friendly products increases. In Europe and Asia, in particular, the threat of impending legislation to regulate waste could have a significant impact on sales of personal care products. Most personal care products include polyolefin-based materials that do not degrade. The challenge is to produce new products that are at parity with or better than the current products in terms of functionality, but that also biodegrade.

One important component of many personal care products is the fluid surge management layer, which typically is placed under the liner and above the superabsorbent layer. The surge layer, herein also referred to as the surge material, manages the flow of fluids to the superabsorbent material. Fluid management is generally measured by the properties of void volume and permeability. If the surge material permeability is too high, the fluid will permeate the superabsorbent material too quickly, causing it to be overwhelmed. If the permeability is too low, the fluid will not progress to the superabsorbent material and can "back-up" into and on the liner. In the meantime, the surge layer should have a sufficient void volume to provide temporary storage for incoming liquid.

The surge layer is usually produced from a bonded carded web (BCW) process. Surge materials currently used employ nondegradable fibers to achieve the desired processability and physical properties, such as void volume and permeability.

A BCW process requires the use of staple cut fibers, usually in a length of approximately 2.5 to 7.6 cm (1 to 3 inches). In order to give the nonwoven web integrity after processing, at least one of the fiber components includes a thermoplastic material that is at least partially melted or softened to bind the web together. Such a component is referred to as a binder fiber. However, the traditional thermoplastic biodegradable fibers have very poor thermal processability for web forming and often undergo severe heat shrinkage. In addition, the current webs require post treatment, such as treatment with a surfactant, to achieve the desired contact angle.

Most slowly biodegradable staple fibers, such as cellulose, are not thermally processable and thus can not be used as a binder fiber.

### Summary of the Invention

The present invention is directed to fibrous nonwoven webs that have suitable fluid management properties to be used as the surge material in personal care articles. In accordance with the present invention a nonwoven web is provided as set forth in claim 1.

The first binder fiber is preferably a multicomponent fiber wherein the majority of the surface component has a melting temperature that is at least about 10 °C less than the melting temperature of the majority of the non-surface component. In a desired embodiment, the surface component is based on poly(lactic acid) (PLA). In a desired embodiment, the first binder fibers are bicomponent fibers and, in a further desired embodiment, are sheath-core fibers with the sheath primarily made of L,D polylactide (LD-PLA), or a polylactide-caprolactone copolymer, and a core primarily made of L-polylactide (L-PLA). The second fiber is a thermoplastic biodegradable fiber, such as, desirably, cellulose acetate.

### Detailed Description of the Invention

The present invention is directed to nonwoven webs for use as the surge layer of personal care articles. The webs include a first fiber, also referred to as the binder fiber, which is a biodegradable thermoplastic fiber that does not undergo severe heat shrinkage. The webs further include a second fiber which is a biodegradable, thermoplastic fiber.

As used herein, the term "biodegradable" is meant to represent that a material degrades from the action of naturally occurring microorganisms such as bacteria, fungi, and algae. As a result, when the nonwoven web, either in the form of fibers or in the form of a nonwoven structure, will be degradable when disposed of to the environment.

The web created by the combination of first and second fibers has appropriate fluid management properties to function as a surge material. The fluid management properties can be measured in terms of permeability and void volume. The surge material described herein has a moderate permeability, in the range of 500-1500 $\mu m^2$, and a high void volume, greater than 25 $cm^3/g$. Permeability is especially important in surge materials, as it controls the rate at which fluid flows to the absorbent layer. If the permeability is too high, as in the case of currently used surge materials, fluid will be released from the surge to the absorbent layer faster than the absorbent structure can take in the liquid. This leads to pooling and potential leakage. If the permeability is too low, the fluid insults will be stored in the surge layer instead of being properly transferred to the absorbent layer. This will also lead to leakage during subsequent liquid insults. Void volume is also important, as it is indicative of the amount of fluid that can be held temporarily in the surge structure. Ideally the void volume should be high in order to hold a larger quantity of liquid. When a sudden fluid surge occurs, it is desired that all the liquid may be held in the surge layer and then slowly metered to the absorbent layer. This requires a high void volume and a moderate permeability.

A biodegradable nonwoven web is disclosed that includes 40% to 95% of a first binder fiber and 60% to 5% of a second thermoplastic biodegradable fiber, wherein the second fibers have a higher melting temperature than the first fibers. All of the fibers are biodegradable. The webs formed according to the invention are biodegradable and, when used as surge layers, demonstrate superior properties to current surge layers.

Because the second thermoplastic fibers have a higher melting temperature than the binder fibers, they may not be affected when the first fibers are bonded. The resulting webs have improved fluid management properties, which can be attributed to a permeability in the range of 500-1500 $\mu m^2$ and a void volume that is greater than 25 $cm^3$/gram.

The biodegradable nonwoven webs of the invention do not require an extra step to create the desired contact angle and thus the wettability is more durable. The general subject of contact angles and the measurement thereof is well known in the art such as, for example, in Robert J. Good and Robert J. Stromberg, Ed., "Surface and Colloid Science - Experimental Methods", Vol. II, (Plenum Press, 1979). Commercial personal care products generally require contact angles that are below 90 degrees, desirably below about 80 degrees, more desirably below about 70 degrees, in order to provide desired liquid transport properties. In general, the lower the contact angle, the better the wettability.

In addition, the webs demonstrate enhanced wicking while still maintaining other fluid management properties.

### I. The Nonwoven Webs

The Binder Fiber

The binder fiber is a biodegradable, thermoplastic fiber that does not undergo severe heat shrinkage. The binder fiber is preferably a multicomponent fiber having a surface component and a non-surface component. The surface component has a melting temperature at least about 10 °C less than the melting temperature of the non-surface component. In one embodiment, the binder fibers include an aliphatic polyester, desirably poly(lactic acid) (PLA).

Poly(lactic acid) is generally prepared by the polymerization of lactic acid. However, it will be recognized by those skilled in the art that a chemically equivalent material may also be prepared by the polymerization of lactide. As such, as used herein, the term "poly(lactic acid)" is intended to represent the polymer that is prepared by either the polymerization of lactic acid or lactide.

Lactic acid and lactide are known to be asymmetrical molecules, having two optical isomers referred to, respectively, as the levorotatory (hereinafter referred to as "L") enantiomer and the dextrorotatory (hereinafter referred to as "D") enantiomer. As a result, by polymerizing a particular enantiomer or by using a mixture of the two enantiomers, it is possible to prepare different polymers that are chemically similar yet which have different properties. In particular, it has been found that by modifying the stereochemistry of a poly(lactic acid) polymer, it is possible to control, for example, the melting temperature, melt rheology, and crystallinity of the polymer. By being able to control such properties, it is possible to prepare a thermoplastic composition and a multicomponent fiber exhibiting desired melt strength, mechanical properties, softness, and processability properties so as to be able to make attenuated, heat-set, and crimped fibers.

Examples of poly(lactic acid) polymers that are suitable for use in the present invention include a variety of poly(lactic acid) polymers from Chronopol Inc., Golden, Colorado. The terms "poly(lactide)", "poly(lactic acid)", and "PLA" are used herein synonymously. use surface fibers

The PLA based fibers described in U.S. Patent No. 5,698,322 can be used. These fibers include a first component having a melting temperature where the first component forms an exposed surface on at least a portion of the multicomponent fiber; and a second component having a melting temperature that is at least about 10 °C greater than the melting temperature exhibited by the first component. This can be achieved, for example, by using as the first component a copolymer of lactide or lactic acid with another comonomer such as caprolactone or another lactide or lactic acid isomer. Two examples are L,D polylactide and polylactide-caprolactone. The melting temperature difference can also be achieved, for example, by using as the first component a first PLA with a L:D ratio, and as the second component a second PLA with a L:D ratio that is greater than the L:D ratio exhibited by the first PLA.

The first component will be included in the multicomponent fiber in an amount that is between greater than 0 to less than 100 weight percent, beneficially between about 5 to about 95 weight percent, more beneficially between about 25 to about 75 weight percent, and suitably between about 40 to about 60 weight percent. The second component will thus be included in the multicomponent fiber in an amount that is between greater than 0 to less than 100 weight percent, beneficially between about 5 to about 95 weight percent, more beneficially between, about 25 to about 75 weight percent, and suitably between about 40 to about 60 weight percent. The weight percent is based upon the total weight of the first component and the second component present in the multicomponent fiber.

In one embodiment, it is desired that the PLA in the second component of the multicomponent fiber have an L:D ratio that is higher than the L:D ratio of the PLA in the first component. It is therefore desired that the poly(lactic acid) polymer in the first component have an L:D ratio that is beneficially less than about 100:0, more beneficially less than about 99.5:0.5, suitably less than about 98:2, and more suitably less than about 96:4, and down to about 90:10, wherein the L: D ratio is based on the moles of the L and D monomers used to prepare the PLA in the first component.

It is desired that the first PLA, having a relatively lower L:D ratio, is present in the first component in an amount that is effective for the first component to exhibit desirable melt strength, fiber mechanical strength, and fiber spinning properties. As such, the first PLA is present in the first component in an amount that is beneficially greater than about 50 weight percent, more beneficially greater than about 75 weight percent, suitably greater than about 90 weight percent, more suitably greater than about 95 weight percent, and most suitably about 100 weight percent, wherein all weight percents are based upon the total weight of the first component.

Similarly, it is desired that the PLA in the second component include less of the D-enantiomer than the PLA in the first component. As such, the PLA in the second component will have an L:D ratio that is greater than the L:D ratio exhibited by the PLA in the first component. It is, therefore, desired that the PLA in the second component have an L: D ratio that is beneficially at least about 96:4, more beneficially at least about 98:2, suitably at least about 99.5:0.5, and more suitably about 100:0, wherein the L:D ratio is based on the moles of the L and D monomers used to prepare the PLA in the second component.

It is desired that the second PLA, having a relatively higher L:D ratio, is present in the second component in an amount that is effective for the second component to exhibit desirable melt strength, fiber mechanical strength, and fiber spinning properties. As such, the second PLA is present in the second component in an amount that is beneficially greater than about 50 weight percent, more beneficially greater than about 75 weight percent, suitably greater than about 90 weight percent, more suitably greater than about 95 weight percent, and most suitably about 100 weight percent, wherein all weight percents are based upon the total weight of the second component.

While each of the first and second components of the multicomponent fiber will substantially include the respective PLAs, such components are not limited thereto and can include other components not adversely effecting the desired properties of the first and the second components and of the multicomponent fiber. Exemplary materials which could be used as additional components would include, without limitation, pigments, antioxidants, stabilizers, surfactants, waxes, flow promoters, solid solvents, particulates, and materials added to enhance processability of the first and the second components. If such additional materials are included in the components, it is generally desired that such additional components be used in an amount that is beneficially less than about 5 weight percent, more beneficially less than about 3 weight percent, and suitably less than about 1 weight percent, wherein all weight percents are based on the total weight amount of the first or the second components.

It is generally desirable that the second component have a melting or softening temperature that is beneficially at least about 10 °C, more beneficially at least about 20 °C, and suitably at least about 25 °C greater than the melting or softening temperature of the first component. Although the absolute melting or softening temperatures of the first and second components are generally not as important as the relative comparison between the two temperatures, it is generally desired that the melting or softening temperatures of the first and second components be within a range that is typically encountered in most useful applications. As such, it is generally desired that the melting or softening temperatures of the first and second components each beneficially be between about 25 °C to about 350 °C, more beneficially be between about 55 °C, to about 300 °C, and suitably be between about 100 °C to about 200 °C.

It is also desired that the PLA in each of the first and second components exhibit weight average molecular weights that are effective for the first and second components to each exhibit desirable melt strength, fiber mechanical strength, and fiber spinning properties. In general, if the weight average molecular weight of a PLA is too high, this represents that the polymer chains are heavily entangled which may result in that component being difficult to process. Conversely, if the weight average molecular weight of a PLA is too low, this represents that the polymer chains are not entangled enough which may result in that component exhibiting a relatively weak melt strength, making high speed processing very difficult. Thus, both the PLAs in each of the first and second component exhibit weight average molecular weights that are beneficially between about 10,000 to about 500,000, more beneficially between about 50,000 to about 400,000, and suitably between about 100,000 to about 300,000. For polymers or polymer blends useful in the present invention, the weight average molecular weight can be determined using a method known to those skilled in the art.

It is also desired that both of the PLAs in each of the first and second components exhibit polydispersity index values that are effective for the first and second components to each exhibit desirable melt strength, fiber mechanical strength, and fiber spinning properties. As used herein, "polydispersity index" is meant to represent the value obtained by dividing the weight average molecular weight of a polymer by the number average molecular weight of the polymer. In general, if the polydispersity index value of a component is too high, the component may be difficult to process due to inconsistent processing properties caused by component segments comprising low molecular weight polymers that have lower melt strength properties during spinning. Thus, the PLAs in each of the first and second components exhibit polydispersity index values that are beneficially between about 1 to about 10, more beneficially between about 1 to about 4, and suitably between about 1 to about 3. For polymers or polymer blends useful in the present invention, the number average molecular weight can be determined using a method known to those skilled in the art.

It is also desired that the PLAs in each of the first and second component exhibit residual monomer percents that are effective for the first and second component to each exhibit desirable melt strength, fiber mechanical strength, and fiber spinning properties. As used herein, "residual monomer percent" is meant to represent the amount of lactic acid or lactide monomer that is unreacted yet which remains entrapped within the structure of the entangled PLAs. In general, if the residual monomer percent of a PLA in a component is too high, the component may be difficult to process due to inconsistent processing properties caused by a large amount of monomer vapor being released during processing that cause variations in extrusion pressures. However, a minor amount of residual monomer in a PLA in a component may be beneficial due to such residual monomer functioning as a plasticizer during a spinning process. Thus, the PLAs in each of the first and second component exhibit a residual monomer percent that are beneficially less than about 15 percent, more beneficially less than about 10 percent, and suitably less than about 7 percent.

It is also desired that the PLAs in each of the first and second components exhibit melt rheologies that are both substantially similar and effective such that the first and second components, when combined, exhibit desirable melt strength, fiber mechanical strength, and fiber spinning properties. The melt rheology of a PLA may be quantified using the apparent viscosity of the PLA and, as used herein, is meant to represent the apparent viscosity of a component at the shear rate and at the temperature at which the component is to be thermally processed as, for example, when the component is processed through a spinneret. Polymers that have substantially different apparent viscosities have been found to not be readily processable. Although it is desired that both the first and second components exhibit apparent viscosities that are substantially similar, it is not critical that such apparent viscosities be identical. Furthermore, it is generally not important as to which of the first or second components has a higher or lower apparent viscosity value. Instead, it is desired that the difference between the apparent viscosity value of the poly(lactic acid) polymer in the first component, measured at the shear rate and at the temperature at which the first component is to be thermally processed, and the apparent viscosity value of the poly(lactic acid) polymer in the second component, measured at the shear rate and at the temperature at which the second component is to be thermally processed, is beneficially less than about 250 Pascal•seconds, more beneficially less than about 150 Pascal·seconds, suitably than about 100 Pascal•seconds, and more suitably less than about 50 Pascal•seconds. In a desired embodiment, the multicomponent fiber is a sheath-core fiber with the sheath primarily made of L,D polylactide (LD-PLA), or a polylactide-caprolactone copolymer, and a core primarily made of "finish the sentence with the already existing on the last line of the document "L-polylactide (L-PLA)." L-polylactide (L-PLA). In a particularly desired embodiment, the sheath is 95:5 L:D polylactide, or a polylactide-caprolactone copolymer, and the core is 100% L-polylactide.

Typical conditions for thermally processing the first and second components include using a shear rate that is beneficially between about 100 seconds$^{-1}$ to about 10000 seconds$^{-1}$, more beneficially between about 500 seconds$^{-1}$ to about 5000 seconds$^{-1}$, suitably between about 1000 seconds$^{-1}$ to about 2000 seconds$^{-1}$, and most suitably at about 1000 seconds$^{-1}$. Typical conditions for thermally processing the first and second components also include using a temperature that is beneficially between about 100 °C to about 500 °C, more beneficially between about 150 °C to about 300 °C, and suitably between about 175 °C to about 250 °C.

Methods for making multicomponent fibers, generally described, involve separately extruding at least two polymers and feeding them to a polymer distribution system where the polymers are introduced into a segmented spinneret plate. The polymers follow separate paths to the fiber spinneret and are combined in a spinneret hole which comprises either at least two concentric circular holes thus providing a sheath/core type fiber or a circular spinneret hole divided along a diameter into at least two parts to provide a side-by-side type fiber. The combined polymer filament is then cooled, solidified, and drawn, generally by a mechanical rolls system, to an intermediate filament diameter and collected. Subsequently, the filament may be "cold drawn" at a temperature below its softening temperature, to the desired finished fiber diameter and crimped or texturized and cut into a desirable fiber length. Multicomponent fibers can be cut into relatively short lengths, such as staple fibers which generally have lengths in the range of about 25 to about 50 millimeters and short-cut fibers which are even shorter and generally have lengths less than about 25 millimeters.

PLA is a typical polyester-based material which often undergoes heat shrinkage during downstream thermal processing. The heat shrinkage mainly occurs due to the thermally induced chain relaxation of the polymer segments in the amorphous phase and incomplete crystalline phase. To overcome this problem, it is generally desirable to maximize the crystallization of the material before the bonding stage so that the thermal energy goes directly to melting rather than to allow for chain relaxation and reordering of the incomplete crystalline structure. One solution to this problem is to subject the material to a heat-setting treatment. As such, when fibers subjected to heat-setting reach a bonding roll, the fibers won't substantially shrink because such fibers are already fully or highly oriented.

Thus it is desired that the multicomponent fibers used in the surge material undergo heat setting. It is desired that such heat-setting occur, when the fibers are subjected to a constant strain of at least 5 percent, at a temperature that is beneficially greater than about 50 °C, more beneficially greater than about 70 °C, and suitably greater than about 90 °C. It is generally recommended to use the highest possible heat-setting temperatures while not sacrificing a fiber's processability. However, too high of a heat-setting temperature as, for example, a temperature close to the melting temperature of the first component of a multicomponent fiber, may reduce the fiber strength and could result in the fiber being hard to handle due to tackiness.

In one embodiment of the present invention, it is desired that the first fiber exhibits an amount of shrinking, at a temperature of about 70 °C, that is beneficially less than about 10 percent, more beneficially less than about 5 percent, suitably less than about 2 percent, and more suitably less than about 1 percent, wherein the amount of shrinking is based upon the difference between the initial and final lengths divided by the initial length multiplied by 100.

The compositions include about 40% to 95% of the binder fiber, wherein all weight percents are based on the total weight amount of the PLA present in the web composition.

### The Second, Biodegradable Fiber

The second fiber is a thermoplastic biodegradable fiber. Thermoplastic biodegradable fibers which are suitable for the composition of the invention include those fibers which are both thermoplastic, that is that flow in the presence of heat so that heat can be used to bond the fibers, and biodegradable. Non-limiting examples of suitable thermoplastic biodegradable fibers include lower alkyl cellulose esters, like cellulose acetate, including cellulose acetate butyrate (CAB), cellulose acetate propionate (CAP), and triacetate cellulose, starch, polyvinyl alcohol (PVA), chitosan, and PHBV (copolymer of polybetahydroxy butyrate and betahydroxyvalerate). Any thermoplastic fiber can be used, providing it has a melting temperature at least about 20 °C higher than the melting temperature of the PLA sheath material.

The use of cellulose acetate offers a number of advantages. In particular, the trilobal shape may enhance fluid management properties. In addition, the very high softening temperature of cellulose acetate, 260 °C, allows it to withstand the bonding conditions used for the thermoplastic binder fibers in this web.

The compositions include about 60% to 5% of the second fiber, wherein all weight percents are based on the total weight amount of the PLA present in the web composition.

## II. Methods of Making the Nonwoven Webs

The nonwoven webs are made by a bonded carded web process. "Bonded carded web" or "BCW" refers to webs that are made from staple fibers which are sent through a combing or carding unit, which separates or breaks apart and aligns the staple fibers in the machine direction to form a generally machine direction-oriented fibrous nonwoven web. Such fibers are usually purchased in bales which are placed in an opener/blender or picker which separates the fibers prior to the carding unit.

The first step in making the nonwoven webs involves massing the fibers and blending them in the desired weight ratio. The fibers are then put through an opening process which opens the tightly grouped fibers and blends the two or more different types of fibers. This opening process consists of a machine which separates the fibers through the use of a picker. These blended fibers are then distributed into a flat layer called a batt. The fiber batt is fed to the carding or combing process which separates and orients the fibers in the machine direction. The card is a large rotating drum with teeth to work the fibers. The carded fibers are then stripped off the card and released in a continuous sheet that is transported by a forming belt.

Once the web is formed, it then is bonded by one or more of several known bonding methods. One such bonding method is powder bonding, wherein a powdered adhesive is distributed through the web and then activated, usually by heating the web and adhesive' with hot air. Another suitable bonding method is pattern bonding, wherein heated calendar rolls or ultrasonic bonding equipment are used to bond the fibers' together, usually in a localized bond pattern, though the web can be bonded across its entire surface if so desired. Another suitable and well-known bonding method, particularly when using conjugate staple fibers, is through-air bonding. Other methods include hydroentanglement, and calendar bonding. To produce the lofty structure desired for surge, through-air bonding is usually the preferred method. Through-air bonding involves subjecting the web to a flow of heated air that penetrates through the web. This air should be hot enough to soften or melt the sheath of the bicomponent binder fibers while leaving the core intact. The desired air temperature will depend upon the type and amounts of the materials used. For example, the temperature should not be so high that it melts the second fiber or the core of the first bicomponent fiber. Moreover, a higher temperature would likely be required to ensure adequate bonding if the composition contains a small amount of the binder fiber. Typical temperatures used for through air bonding are within the range of about 270 °F to 330 "F (132 °C to 166°C).

The webs include from about 40% to 95% of the binder fiber, and most desirably about 50 to 90%. The webs include about 60% to 5% of the second fiber and most desirably about 50 to 10%.

**III. Methods of Using the Nonwoven Webs**

The nonwoven webs of the present invention are suited for use as the surge layer in disposable products including disposable absorbent products such as diapers, adult incontinent products, and bed pads; in catamenial devices such as sanitary napkins, and tampons; and other absorbent products such as wipes, bibs, wound dressings, and surgical capes or drapes.

A typical disposable absorbent product includes a liquid-permeable topsheet, a backsheet attached to the liquid-permeable topsheet, and an absorbent structure positioned between the liquid-permeable topsheet and the backsheet. The surge layer is typically positioned between the topsheet and the absorbent structure. Exemplary disposable absorbent products are generally described in U.S. Patent No. 4,710,187; U.S. Patent No. 4,762,521; U.S. Patent No. 4,770,656; and U.S. Patent No. 4,798,603.

The invention is further illustrated by the following examples, which are not to be construed in any way as imposing limitations upon the scope thereof.

**Examples**

Test Methods

Basis weight was determined by cutting a 10cm by 10cm (4 inch by 4 inch) square of nonwoven material and weighing it on an analytical balance. Five such squares were cut and the results averaged to arrive at the basis weight.

Density was determined by dividing the basis weight by the thickness of the web. The thickness was determined using a Digimatic Indicator with an applied pressure of 345Pa (0.05 psi).

Void volume was calculated as the inverse of density.

Permeability was calculated using the PERMIX equation:

$$\kappa = C_1 \cdot r^2 \cdot (1-\phi)[(\phi/(1-\phi))(sat/sat_o)]^{C2}$$

where r is the fiber radium, sat is the level of saturation, and $sat_o$ is 1(100% saturation). $C_1$ and $C_2$ depend on the geometry of the fiber and the orientation within the web, typical values are 0.075 and 2.5, respectively. $\phi$ is the void volume fraction (the percent open area of the web structure). A high permeability number indicates little resistance is being encountered and the liquid is flowing quickly, easily, and relatively uncontrolled.

Bulk recovery is a measure of the web's ability to return to its original condition following an applied pressure. Using the Digimatic Indicator, the thickness of the sample at applied pressures of 345Pa (0.05 psi), then 1379Pa (0.2 psi), and then 345Pa (0.05 psi) was measured. The ratio of the thickness at the final measurement to the initial thickness, multiplied by 100 to yield percent, is the Percent Bulk Recovery. This is particularly important for applications where a product may be subjected to the almost the full weight load of the user, for example in a baby diaper or adult incontinence care product.

Web Contact Angle was determined by weighting the component contact angles based on the mass percentage of each component.

Fluid Intake and Flowback Evaluation (FIFE) testing was used to determine the absorbency or intake time and flowback. A Maser-Flex Digi-Static Automatic Dispensing system was supplied with saline colored with a small amount of FD&C blue dye, set to provide 80 ml insults, and dispensed several times to eliminate any air bubbles. The product samples, infant diapers, were prepared without elastic so that they would easily lie flat. Two 8.9cm by 30.5cm (3.5 inch by 12 inch) blotter paper samples were weighed. These papers were placed on the first FIFE board, a simple board with a 7.6cm by 15.2cm (3 inch by 6 inch) raised platform in the middle. The blotter papers were aligned so that they ran lengthwise along either side of the raised platform. The diaper was then aligned so that the area to be insulted was carefully centered on the raised platform, with the top sheet facing up, such that there were no visible wrinkles in the nonwoven top sheet. The second FIFE board was then placed on top of the product. The second FIFE board is a flat board intersected by a hollow cylinder, protruding only from the top side of the board. The circular region created where the cylinder intersected the flat plane of the board was hollow. The inner diameter of the cylinder was 5.1 cm. A funnel with an inner diameter of 7 mm at the short end was placed in the cylinder. The fluid was then dispensed by the pump directly into the funnel. The intake time was recorded by stopwatch from the time the fluid hit the funnel to the moment no fluid was visible on the specimen surface. The blotter papers were checked for product leakage and, if any occurred, the weight of the blotter papers was measured to determine the quantity of fluid that leaked. In the described testing, no leakage occurred. Approximately one minute elapsed before the second insult was applied in the same manner.

Following absorption of the second insult, the top FIFE board was removed and the sample was placed liner side up on a Saturated Capacity Tester. The specimen sat for about 1 minute and then two tared 30.5cm (12 inch) blotter papers were placed, one on top of the other, over the target area. This was then covered with a latex rubber sheet and the vacuum valve adjusted to read 345Pa (0.5 psi). After 2 minutes the latex was lifted to release the pressure and the specimen sat for 1 minute. The wet blotter papers were then weighed to determine the amount of liquid they contained. The specimen was returned to the FIFE boards in the initial configuration and a third insult applied in the manner of the first two. The procedure of placing the sample on the Saturated Capacity Tester with the tared blotter paper and applying vacuum was repeated. The amount of liquid absorbed by the blotter paper after the third insult is the Flowback value.

Web Production

Bicomponent PLA fibers were made at Chisso of Mariyama, Japan. The fibers were bicomponent fibers with a sheath of 95:5 L-D polylactide and a core of 100% L-polylactide. The fibers were heat-set, crimped, and cut into a length of 3.18cm (1.25 inch). Trilobal cellulose acetate fibers obtained from Celanese Acetate were used. These fibers were selected for their biodegradability and excellent tenacity. The tenacity ranges from about 1.2 to 1.4 G/D at standard conditions and 0 to 1.0 G/D when wet and at 21°C (70 °F). The elongation at break under standard conditions is 25 to 45% and is 35 to 50 % when wet and at 21°C (70 °F). Table 1 summarizes some of the properties of the fibers that were used.

**Table 1. Starting Materials Information**

| Fiber | Length (cm) [(inches)] | Denier | Crimp Level (crimps/cm) [(crimps/inch)] | Surface Melting Temperature (°C) |
|---|---|---|---|---|
| Cellulose Acetate | 3·81 [1.5] | 1.7 | 4-6 [10-15] | 260 |
| Bicomponent PLA | 3·18 [1.25] | 4.0 | 6-8 [15-20] | 145 |

The fibers were massed and blended in the weight ratios indicated in Table 2. The fibers were then put through an opening process in a machine which separates the fibers through the use of a picker. The blended fibers were then distributed into a batt. The fiber batt was fed to the carding drum. The carded fibers were then stripped off the card and released in a continuous sheet that was transported by a forming belt. This sheet was then bonded using through-air bonding. Table 2 summarizes the samples produced and process conditions used.

**Table 2. Samples and Process Conditions**

| Sample # | Fiber #1 | Fiber #2 | Weight Ratio of Fiber 1 to Fiber 2 | Number of Passes Through Opener | Through-Air Bonder Temperature (°C) |
|---|---|---|---|---|---|
| 1 | Bicomponent PLA | | 100:0 | 5 | 168 |
| 2 | Bicomponent PLA | Cellulose Acetate | 70:30 | 5 | 168 |
| 3 | Bicomponent PLA | Cellulose Acetate | 70:30 | 5 | 143 |

Sample 2 was a hard, lumpy web due to being overbonded. Sample 3 was a soft, strong web and was further evaluated.

Web Testing

The physical properties of the web produced in Sample #3 above, bicomponent PLA fibers alone, and a currently used surge material are given in Table 3. The currently used surge material was a bonded carded web, having a basis weight of 2.5 osy, consisting of a polyester staple fiber and a sheath/core bicomponent fiber in a ratio of 60:40, made by Kimberly-Clark Corporation. The sheath was polyethylene and the core was polypropylene.

**Table 3. Physical Properties**

| Properties | Current Surge Material | 100% Bicomponent PLA | Sample #3 |
|---|---|---|---|
| Basis Weight (gsm) | 87.45 | 103 | 88.16 |
| Void Volume ($cm^3/g$) | 26.04 | 14.30 | 31.62 |
| Permeability ($\mu m^2$) | 2078 | 402.3 | 1231 |
| Bulk Recovery (%) | 88.9 | 98.5 | 97.6 |
| Web Contact Angle (degrees) | 76 | 85 | 74 |
| No Load Horizontal Saturation Capacity (grams) | 30.17 | ----- | 30.41 |
| Vertical Wicking (cm) | 0.5 | ----- | 0.94 |
| Flowback (grams) | 16.1 | ----- | 13.1 |

The moderate permeability and high void volume of the web of Sample #3 provides improved fluid transport properties. This is evidenced by the increase in vertical wicking over the control. The Sample #3 web had a contact angle that is very similar to the current surge material. It should be noted that in the case of Sample #3, the low contact angle was due to the intrinsic properties of the fibers comprising the web, whereas the current surge material requires a surfactant treatment to achieve the contact angle of 76. The vertical wicking of the current surge was only 0.5 cm, while Sample #3 demonstrated vertical wicking of almost 1 cm. Because the web contact angles were so similar, this difference in wicking can be attributed to the unique physical structure of the invented surge material. The vertical wicking test indicated that there was improved fluid transport in the web of Sample #3.

It is noted that Sample #3 had a No Load Saturation Capacity at parity with the control. This is the amount of fluid that the nonwoven can hold under no applied pressure.

In terms of flowback, it is noted that Sample #3 had a lower flowback value than the current surge material. This is important, because in personal care applications, such as an infant care diaper, it is desired to minimize the amount of fluid in contact with the body. A surge layer can be put under pressure during normal wear and fluid flowback is undesirable.

The above description is intended to be illustrative and not restrictive. Many embodiments will be apparent to those of skill in the art upon reading the above description. The scope of the invention should, therefore, be determined not with reference to the above description, but should instead be determined with reference to the appended claims, along with the full scope of equivalents to which such claims are entitled.

**Claims**

1. A nonwoven web having a permeability within the range of about 500 to about 1500 $\mu m^2$ and a void volume that is greater than about 25 $cm^3$/gram, wherein the web includes a first biodegradable binder fiber that does not undergo severe heat shrinkage, and a second biodegradable, thermoplastic fiber, the web comprising from about 40% to 95% of the first fiber and from about 60% to 5% of the second fiber, wherein the second fiber has a higher melting temperature than the first fiber.

2. The nonwoven web of claim 1, wherein the first fiber is a multicomponent fiber including poly(lactic acid) (PLA).

3. The nonwoven web of claim 2, wherein the multicomponent fiber comprises a surface component and a non-surface component and the surface component has a melting temperature at least about 10 °C less than the melting temperature of the non-surface component.

4. The nonwoven web of claim 3, wherein the second thermoplastic fiber has a melting temperature at least about 20 °C higher than the melting temperature of the first fiber surface component.

5. The nonwoven web of claim 3, wherein the surface component comprises L,D-polylactide (LD-PLA), or a polylactide-caprolactone copolymer.

6. The nonwoven web of claim 3, wherein the surface component comprises L,D-polylactide (LD-PLA), the non-surface component comprises polylactide, and the surface component has a lower L:D ratio than the non-surface component.

7. The nonwoven web of claim 2, wherein the first fiber is a bicomponent sheath/core fiber.

8. The nonwoven web of claim 7, wherein the sheath is 95:5 L:D polylactide, or a polylactide-caprolactone copolymer, and the core is 100% L-polylactide.

9. The nonwoven web of any preceding claim, wherein the first fiber exhibits an amount of shrinkage, at a temperature of about 70°C, that is less than about 10 percent.

10. The nonwoven web of any preceding claim, wherein the second fiber is selected from the group consisting of lower alkyl cellulose esters, starch, polyvinyl alcohol (PVA), chitosan, and PHBV (copolymer of polybetahydroxy butyrate and betahydroxyvalerate).

11. The nonwoven web of claim 10, wherein the lower alkyl cellulose ester is cellulose acetate.

12. The nonwoven web of any preceding claim, further having a contact ang!e less than 80 degrees, and wherein the contact angle is due to intrinsic properties of the fibers.

13. The nonwoven web of any preceding claim, wherein the web is produced by a bonded carded web process using through-air bonding.

14. An absorbent article comprising a surge layermade from the nonwoven web of any preceding claim.

15. The absorbent article of claim 14, comprising a liquid-permeable topsheet, a backsheet attached to the liquid-permeable topsheet, an absorbent structure positioned between the liquid-permeable topsheet and the backsheet, and wherein the surge layer is positioned between the topsheet and the absorbent structure.

**Patentansprüche**

1. Vliesstofflage mit einer Permeabilität im Bereich von ungefähr 500 bis ungefähr 1500 $\mu m^2$ und einem Leerraumvolumen, welches größer als ungefähr 25 cm$^3$/Gramm ist, wobei die Lage eine erste biologisch abbaubare Bindefaser, welche keine starke Wärmeschrumpfung durchmacht, und eine zweite biologisch abbaubare thermoplastische Faser einschließt, wobei die Lage ungefähr 40 % bis 95 % der ersten Faser und ungefähr 60 % bis 5 % der zweiten Faser umfasst, wobei die zweite Faser eine höhere Schmelztemperatur aufweist als die erste Faser.

2. Vliesstofflage nach Anspruch 1, wobei die erste Faser eine Mehrkomponentenfaser ist, die Polymilchsäure (PLA) einschließt.

3. Vliesstofflage nach Anspruch 2, wobei die Mehrkomponentenfaser eine Oberflächenkomponente und eine Nicht-Oberflächenkomponente umfasst und die Oberflächenkomponente eine Schmelztemperatur aufweist, die wenigstens ungefähr 10 °C geringer ist als die Schmelztemperatur der Nicht-Oberflächenkomponente.

4. Vliesstofflage nach Anspruch 3, wobei die zweite thermoplastische Faser eine Schmelztemperatur aufweist, die wenigstens ungefähr 20 °C höher ist als die Schmelztemperatur der ersten Faseroberflächenkomponente.

5. Vliesstofflage nach Anspruch 3, wobei die Oberflächenkomponente L,D-Polylactid (LD-PLA) oder ein Polylactid-Caprolacton-Copolymer umfasst.

6. Vliesstofflage nach Anspruch 3, wobei die Oberflächenkomponente L,D-Polylactid (LD-PLA) umfasst, die Nicht-Oberflächenkomponente Polylactid umfasst und die Oberflächenkomponente ein niedrigeres L:D Verhältnis aufweist als die Nicht-Oberflächenkomponente.

7. Vliesstofflage nach Anspruch 2, wobei die erste Faser eine Bikomponenten-Mantel/Kern-Faser ist.

8. Vliesstofflage nach Anspruch 7, wobei der Mantel 95:5 L:D Polylactid oder ein Polylactid-Caprolacton-Copolymer ist und der Kern 100 % L-Polylactid ist.

9. Vliesstofflage nach einem vorangehenden Anspruch, wobei die erste Faser ein Maß der Schrumpfung bei einer Temperatur von ungefähr 70 °C aufweist, das weniger als ungefähr 10 % beträgt.

10. Vliesstofflage nach einem vorangehenden Anspruch, wobei die zweite Faser ausgebildet ist aus der Gruppe bestehend aus Niederalkylcelluloseestern, Stärke, Polyvinylalkohol (PVA), Chitosan und PHBV (Copolymer von Polybetahydroxybutyrat und Betahydroxyvalerat).

11. Vliesstofflage nach Anspruch 10, wobei der Niederalkylcelluloseester Celluloseacetat ist.

12. Vliesstofflage nach einem vorangehenden Anspruch, die außerdem einen Kontaktwinkel von weniger als 80 Grad aufweist und wobei der Kontaktwinkel auf den Fasern innewohnende Eigenschaften zurückzuführen ist.

13. Vliesstofflage nach einem vorangehenden Anspruch, wobei die Lage durch ein Verfahren für bondierte-kardierte Lagen unter Verwendung der Through-air-bonding-Technik hergestellt ist.

14. Absorptionsfähiger Artikel umfassend eine Pufferschicht, die aus der Vliesstofflage nach einem vorangehenden Anspruch hergestellt ist.

15. Absorptionsfähiger Artikel nach Anspruch 14, umfassend eine flüssigkeitsdurchlässige Decklage, eine an der flüssigkeitsdurchlässigen Decklage angebrachte rückseitige Lage und eine absorptionsfähige Struktur, die zwischen der flüssigkeitsdurchlässigen Decklage und der rückseitigen Lage angeordnet ist, wobei die Pufferschicht zwischen der Decklage und der absorptionsfähigen Struktur angeordnet ist.


**Revendications**

1. Voile non-tissé ayant une perméabilité comprise dans la gamme allant d'environ 500 à environ 1500 $\mu m^2$ et un volume de vides supérieur à environ 25 $cm^3$/gramme, dans lequel le voile inclut une première fibre liante biodégradable qui ne subit pas de sérieux retrait thermique, et une seconde fibre thermoplastique biodégradable, le voile renfermant d'environ 40 % à 95 % de la première fibre et d'environ 60 % à 5 % de la seconde fibre, la seconde fibre ayant une température de fusion plus élevée que la première fibre.

2. Voile non-tissé selon la revendication 1, dans lequel la première fibre est une fibre multicomposée incluant du poly (acide lactique) (PLA).

3. Voile non-tissé selon la revendication 2, dans lequel la fibre multicomposée comprend un composant superficiel et un composant non superficiel, et le composant superficiel a une température de fusion d'au moins environ 10°C inférieure à la température de fusion du composant non superficiel.

4. Voile non-tissé selon la revendication 3, dans lequel la seconde fibre thermoplastique a une température de fusion supérieure d'au moins 20°C à la température de fusion du composant superficiel de la première fibre.

5. Voile non-tissé selon la revendication 3, dans lequel le composant superficiel comprend du L,D-polylactide (LD-PLA) ou un copolymère polylactide-caprolactone.

6. Voile non-tissé selon la revendication 3, dans lequel le composant superficiel comprend du L,D-polylactide (LD-PLA), le composant non superficiel comprend du polylactide, et le composant superficiel a un rapport L:D inférieur à celui du composant non superficiel.

7. Voile non-tissé selon la revendication 2, dans lequel la première fibre est une fibre bicomposée écorce/noyau.

8. Voile non-tissé selon la revendication 7, dans lequel l'écorce est en polylactide L:D 95:5, ou en un copolymère polylactide-caprolactone, et le noyau est formé à 100 % de L-polylactide.

9. Voile non-tissé selon l'une quelconque des revendications précédentes, dans lequel la première fibre manifeste, à une température d'environ 70°C, une quantité de retrait qui est inférieure à environ 10 %.

**10.** voile non-tissé selon l'une quelconque des revendications précédentes, dans lequel la seconde fibre est sélectionnée dans le groupe consistant en les esters d'(alkyle inférieur) cellulose, l'amidon, le poly(alcool vinylique) (PVA), le chitosane et le PHBV [copolymère du poly(bêtahydroxybutyrate) et du bêtahydroxyvalérate].

**11.** Voile non-tissé selon la revendication 10, dans lequel l'ester d'(alkyle inférieur)cellulose est l'acétate de cellulose.

**12.** Voile non-tissé selon l'une quelconque des revendications précédentes, ayant en outre un angle de contact inférieur à 80 degrés, ledit angle de contact étant du aux propriétés intrinsèque des fibres.

**13.** Voile non-tissé selon l'une quelconque des revendications précédentes, dans lequel le voile est produit par un procédé de cardage-liaison de voile utilisant une liaison par soufflage transversal d'air.

**14.** Article absorbant comprenant une couche de gestion de l'afflux faite du voile non-tissé selon l'une quelconque des revendications précédentes.

**15.** Article absorbant selon la revendication 14, comprenant une feuille supérieure perméable aux liquides, une feuille-dossier fixée à la feuille supérieure, une structure absorbante positionnée entre la feuille supérieure perméable aux liquides et la feuille-dossier, la feuille de gestion de l'afflux étant positionnée entre la feuille supérieure et la structure absorbante.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5698322 A
- US 4710187 A
- US 4762521 A
- US 4770656 A
- US 4798603 A

**Non-patent literature cited in the description**

- Surface and Colloid Science - Experimental Methods. Plenum Press, 1979, vol. II